# EUROPÄISCHE PATENTSCHRIFT

(11) **EP 2 583 606 B1**
(45) Veröffentlichungstag und Bekanntmachung des Hinweises auf die Patenterteilung: **29.10.2014**
(21) Anmeldenummer: 12006765.7
(22) Anmeldetag: 27.09.2012
(51) Int. Cl.: A47L 9/14, D04H 3/007, D01F 1/10, A61F 13/00, A23J 1/14, A23K 1/14, C10L 1/18

(54) **Staubsaugerfilterbeutel mit Geruchsabsorber und Verfahren zur Herstellung eines Spinnvlieses**
Vacuum cleaner bag with deodorizer and method of making a meltspun nonwoven
Sac d' aspirateur et méthode de fabrication d'un non-tissé melt-spun

(30) Priorität: 21.10.2011 DE 102011116554
(43) Veröffentlichungstag der Anmeldung: 24.04.2013
(73) Patentinhaber: Innovatec Microfibre Technology GmbH & Co. KG, 53840 Troisdorf (DE)
(72) Erfinder: Waeteraere, Michael, 53797 Lohmar (DE); Fengler, Eduard, 53844 Troisdorf (DE)
(74) Vertreter: Patentanwaltskanzlei Methling

(56) Entgegenhaltungen:
- EP-A1- 1 674 014
- EP-A1- 1 972 247
- WO-A1-2007/053790
- DE-A1- 3 808 114
- JP-A- 3 279 409
- US-A1- 2005 255 078

## Beschreibung

Die Erfindung betrifft einen Staubsaugerfilterbeutel, welcher aus einem mehrlagigen Verbundmaterial aufweisend mehrere Filterlagen gebildet ist und einen eingefassten Anschlussbereich aufweist, wobei zumindest eine Filterlage durch ein Feinfaserspinnvlies auf Basis eines Polymers, insbesondere Polyolefin, insbesondere Polypropylen, gebildet ist, wobei zur Faserherstellung der Polymer als Rohmaterial in einem Extruder erhitzt und unter hohem Druck durch eine Spinndüse gepresst wird.

Ferner betrifft die Erfindung ein Verfahren zur Herstellung eines Spinnvlieses auf Basis eines Polymers, insbesondere Polyolefin, insbesondere Polypropylen, wobei zur Faserherstellung der Polymer als Rohmaterial in einem Extruder erhitzt und unter hohem Druck durch eine Spinndüse gepresst wird.

Vliesstoff oder kurz Vlies ist ein textiles Flächengebilde aus einzelnen Fasern. Im Sinne der Erfindung werden die Begriffe Feinfaserspinnvlies, Spinnvlies, Vlies oder Vliesstoff unabhängig von dem konkreten Herstellungsverfahren synonym verwendet und es sind alle nichtgewebten Stoffe umfasst, die in der englischen Sprache als sogenannte "nonwoven" bezeichnet werden. Ein Vlies besteht aus lose zusammen liegenden Fasern, welche noch nicht miteinander verbunden sind. Die Festigkeit eines Vlieses beruht nur auf der fasereigenen Haftung, kann aber durch Aufarbeitung beeinflusst werden. Um dem Vlies besondere Festigkeitseigenschaften zu verleihen, kann es weiter verfestigt werden, wofür verschiedene Methoden angewandt werden können, insbesondere das Kalandrieren.

Bei einem ersten bekannten Verfahren der Faserherstellung wird ein Polymer in einem Extruder erhitzt und auf einen hohen Druck gebracht. Das Polymer wird in genauer Dosierung mittels der Spinnpumpen durch eine Matrize, die so genannte Spinndüse gepresst. Das Polymer tritt aus der Düsenplatte als feiner Faden noch in geschmolzener Form aus. Durch einen Luftstrom wird es abgekühlt und noch aus der Schmelze gestreckt. Der Luftstrom befördert die Fäden auf ein Förderband das als Sieb ausgebildet ist. Durch eine Absaugung unter dem Siebband werden die Fäden fixiert. Dieses Faden- bzw. Fasergelege ist ein Wirrlagen-Vlies, das verfestigt werden muss. Die Verfestigung kann durch zwei beheizte Kalanderwalzen durch das sogenannte Kalandrieren oder durch einen Dampfstrom erfolgen.

Bei dem sogenannten Meltblown-Verfahren wird zur Faserherstellung ebenfalls ein Polymer in einem Extruder erhitzt und auf einen hohen Druck gebracht. Das Polymer wird in genauer Dosierung mittels der Spinnpumpen durch eine Matrize, die so genannte Spinndüse gepresst. Nach dem Austritt durch die Düsenspitze wird das Polymer durch komprimierte Prozess-Heißluft gestreckt, d.h. mittels eines Hochgeschwindigkeitsheißluftstromes gestreckt. Das entstehende Mikrofaservlies wird auf einem luftdurchlässigen Siebband abgelegt. Zur Herstellung von Laminaten kann die Meltblown-Anlage um eine Abrollstation vor und hinter der Düse erweitert sein. Ein Kalander verbindet die zugeführten Materialien. So werden ein-, zwei- und mehrlagige Stoffe produziert.

Derartige Feinfaserspinnvliese oder einfach Spinnvliese (englisch allgemein: Nonwoven) sind bekannt und werden insbesondere als Filter und hier ferner in Staubsaugerfilterbeuteln eingesetzt. Um unangenehmer Gerüche zu unterdrücken ist es bekannt, Duftkapseln in den Staubsauger einzulegen. Dies führt zu einer Vermischung des unangenehmen Geruches mit dem Aroma der Duftkapsel.

Nachteilig bei den bekannten Vliesstoffen ist es, dass diese nicht geeignet sind, unangenehme Gerüche zu binden und es ferner sehr aufwändig ist, bei dem Einsatz solcher Vliesstoffe als Staubsaugerflterbeutel zusätzlich jeweils eine Duftkapsel in den Staubsauger einzulegen.

Die Aufgabe der Erfindung ist es, einen Staubsaugerfilterbeutel derart weiterzubilden, dass dieser geeignet ist, unangenehme Gerüche zu binden und eine einfache Handhabung ohne den Einsatz von Duftkapseln oder dergleichen gestattet.

Eine weitere Aufgabe der Erfindung ist es, ein Herstellungsverfahren zur Herstellung eines Feinfaserspinnvlieses anzugeben, um ein Feinfaserspinnvlies zu erzeugen, welches geeignet ist, unangenehme Gerüche zu binden.

Diese Aufgabe wird erfindungsgemäß durch einen Staubsaugerfilterbeutel gemäß Anspruch 1 und das Herstellungsverfahren gemäß Anspruch 7 gelöst. Vorteilhafte Weiterbildungen der Erfindung sind in den jeweiligen abhängigen Ansprüchen angegeben.

Im Sinne der Erfindung werden die Begriffe Feinfaserspinnvlies, Spinnvlies, Vlies oder Vliesstoff unabhängig von dem konkreten Herstellungsverfahren oder der Weiterverarbeitung der Fasern synonym verwendet und es sind alle nichtgewebten Stoffe umfasst, die in der englischen Sprache als sogenannte "Nonwoven" bezeichnet werden, insbesondere sind alle durch Schmelzspinnen hergestellten Fasern und hieraus erzeugte nichtgewebte Stoffe umfasst.

Besonders Vorteilhaft bei dem erfindungsgemäßen Staubsaugerfilterbeutel, welcher aus einem mehrlagigen Verbundmaterial aufweisend mehrere Filterlagen gebildet ist und einen eingefassten Anschlussbereich aufweist, wobei zumindest eine Filterlage durch ein Feinfaserspinnvlies auf Basis eines Polymers, insbesondere Polyolefin, insbesondere Polypropylen, gebildet ist, wobei zur Faserherstellung der Polymer als Rohmaterial in einem Extruder erhitzt und unter hohem Druck durch eine Spinndüse gepresst wird, ist es, dem Rohmaterial des Vlieses vor dem Extrudieren oder während des Extrudierens im Extruder ein Anteil von 1 % bis 50 % eines Kunststoffadditivs enthaltend Zinksalz der Ricinolsäure beigemischt wird, so dass das Material der Fasern des Vlieses Zinksalz der Ricinolsäure aufweist, wobei das Material der Fasern des Vlieses einen Gewichtsanteil von bis zu 10 % Zinksalz der Ricinolsäure aufweist.

Als Rohmaterial zur Faserherstellung können dabei sowohl natürliche Polymere, wie beispielsweise Cellulose, oder auch synthetische Polymere, insbesondere Polypropylen zum Einsatz kommen.

Erfindungsgemäß weist das Fasermaterial des Spinnvlieses ein Geruchsabsorberadditiv auf. Dabei ist Zinksalz der Ricinolsäure bzw. Zink-Ricinoleat als Geruchsabsorber besonders gut geeignet. Der verwendete Wirkstoff, d.h. das Zinksalz der Ricinolsäure bindet die Verursacher der schlechten Gerüche, wie Stickstoff- und Schwefelverbindungen fest in einer Matrix ein und verhindert so, dass Gerüche entstehen und nach außen freigesetzt werden können.

Dabei wird dem Rohmaterial zur Faserherstellung des Vlieses vor dem Extrudieren im Extruder an Anteil von 1 % bis 50 % eines Kunststoffadditivs enthaltend Zinksalz der Ricinolsäure beigemischt. Insbesondere kann dieses Kunststoffadditiv einen Gewichtsanteil von 10 % bis 20 %, insbesondere von 15 % Zinksalz der Ricinolsäure aufweisen.

Alternativ besteht auch die Möglichkeit, dass dem Rohmaterial zur Faserherstellung des Vlieses während des Extrudierens im Extruder ein Anteil von 1 % bis 50 % eines Kunststoffadditivs enthaltend Zinksalz der Ricinolsäure beigemischt wird, wobei das Kunststoffadditiv einen Gewichtsanteil von 10 % bis 20 %, insbesondere 15 % Zinksalz der Ricinolsäure aufweist.

Es besteht somit die Möglichkeit, ein Kunststoffadditiv bereitzustellen, welches einen höheren Gewichtsanteil an Zinksalz der Ricinolsäure aufweist und dieses dem Rohmaterial zur Faserherstellung beizumischen. Dabei besteht die Möglichkeit, die Beimischung vor der Zufuhr zum Extruder durchzuführen. Alternativ besteht auch die Möglichkeit, die Beimischung des Kunststoffadditivs im Extruder vorzunehmen, indem dem Extruder ein Anteil des Rohmaterials zur Faserherstellung zugeführt wird und gleichzeitig ein geringerer Massenstrom an beizumischendem Kunststoffadditiv dem Extruder zugeführt wird.

Eingesetzt werden können die erfindungsgemäßen Feinfaserspinnvliese dabei universell, insbesondere in solchen Anwendungen, in denen sie als Filtermaterial, in Filtern und Filtereinsätzen wie beispielsweise als Bestandteil von Staubsaugerfilterbeuteln eingesetzt werden und generell in Anwendungen, bei denen es gewünscht ist, dass unangenehme Gerüche gebunden werden.

Vorzugsweise weist das Material der Fasern des Vlieses des Staubsaugerfilterbeutels einen Gewichtsanteil von 0,1 % bis zu 10 %, insbesondere von 0,5 % bis zu 1,5 %, insbesondere von bis zu 2 % oder bis zu 3 % oder bis zu 4 % oder bis zu 5 % oder bis zu 6 % oder bis zu 7 % oder bis zu 8 % oder bis zu 9 % Zinksalz der Ricinolsäure auf.

Es hat sich gezeigt, dass ein solcher Gewichtsanteil von Zinksalz der Ricinolsäure an dem Material der Fasern des Vlieses des Staubsaugerfilterbeutels eine besonders gute Eignung des Vlieses zur Absorbierung unangenehmer Gerüche bewirkt. Ferner hat sich gezeigt, dass die geruchsabsorbierende Wirkung mit steigendem Anteil an Zinksalz der Ricinolsäure an dem Material der Fasern des Vliesstoffes ansteigt. Mit steigendem Anteil an Zinksalz der Ricinolsäure an dem Material der Fasern des Vliesstoffes steigt sowohl die Wirksamkeit der Bindung unangenehmer Gerüche, als auch die mögliche Einsatzdauer des Vliesstoffes, bevor die Geruchsbindungswirkung merklich absinkt.

In einer bevorzugten Weiterbildung der Erfindung weist das Material der Fasern des Vlieses Aromastoffe auf, insbesondere einen Gewichtsanteil von bis zu 1,5 % Aromastoffe.

Durch die Einbringung von Aromastoffen zusätzlich zu dem geruchsbindendem Zinksalz der Ricinolsäure kann ein Vliesstoff erzeugt werden, der einerseits unangenehme Gerüche durch das Binden der die Gerüche erzeugenden Stickstoff- und Schwefelverbindungen bindet, und gleichzeitig durch die im Material der Fasern des Vliesstoffes enthaltenen Aromastoffe ein angenehmes Aroma verströmt. Dabei können beliebige Aromen verwendet werden.

Vorzugsweise erfolgt nach dem Extrudieren im Extruder und dem Spinnen der Fasern eine Verfestigung der Fasern durch Kalandrieren zwischen zwei Kalanderwalzen und/oder durch Kleben und/oder durch Ultraschallverschweißung. Dabei sind die Kalanderwalzen beheizt und die Fasern werden angeschmolzen, so dass das Feinfaserspinnvlies verfestigt wird und die gewünschten mechanischen Eigenschaften aufweist. Bei der Verfestigung durch einen Kalander ist meist eine der beiden Walzen mit einer Gravur versehen. An den Kontaktpunkten verschmelzen die Fasern und bilden somit einen festen Vliesstoff, der die gewünschten mechanischen Eigenschaften aufweist und dazu führt, dass der Vliesstoff für eine Vielzahl von Verwendungen, wie beispielsweise als Filtermaterial geeignet ist.

Besonders vorteilhaft bei dem erfindungsgemäßen Staubsaugerfilterbeutel, welcher aus einem mehrlagigen Verbundmaterial aufweisend mehrere Filterlagen gebildet ist und einen eingefassten Anschlussbereich aufweist, ist es, dass zumindest eine Filterlage durch ein derartiges Spinnvlies gebildet ist.

Hierdurch ist der Staubsaugerfilterbeutel insbesondere geeignet, unangenehme Gerüche zu binden, d.h., dass der Staubsaugerbeutel nach der Benutzung weniger unangenehme Gerüche freisetzt, als dies nach dem vorbekannten Stand der Technik der Fall ist. Die Bindung der Gerüche beruht auf der beschriebenen Wirkung des Zinksalzes der Ricinolsäure, Stickstoff- und Schwefelverbindungen in einer Matrix fest einzubinden und hierdurch zu verhindern, dass Gerüche entstehen und nach außen dringen.

Vorzugsweise weist das Verbundmaterial des Staubsaugerfilterbeutels zumindest eine Grobfilterlage und eine Feinfilterlage auf.

Der Anschlussbereich des Staubsaugerfilterbeutels, welcher zum Einsetzen in den Staubsauger und zum Anschluss an den Staubsaugerschlauch dient, kann durch einen Kunststoffeinsatz oder einen Einsatz aus Fasermaterial, insbesondere Pappe, oder einen Verbund aus Kunststoff und Fasermaterial gebildet sein, wobei dieser Anschlussbereich an den das den Beutel bildende Verbundmaterial angeklebt und/oder kraftschlüssig geklemmt und/oder angeschweißt ist.

Besonders vorteilhaft bei dem Verfahren zur Herstellung eines Spinnvlieses auf Basis eines Polymers, insbesondere Polyolefin, insbesondere Polypropylen, wobei zur Faserherstellung der Polymer als Rohmaterial in einem Extruder geschmolzen und unter hohem Druck durch eine Spinndüse gepresst wird, ist es, dass dem Rohmaterial zur Faserherstellung während des Extrudierens im Extruder ein Anteil von 1 % bis 50 % eines Kunststoffadditivs enthaltend Zinksalz der Ricinolsäure beigemischt wird, insbesondere ein Kunststoffadditiv mit einem Gewichtsanteil von 10 % bis 20 %, insbesondere 15 % Zinksalz der Ricinolsäure.

Bei einem weiteren Verfahren zur Herstellung eines Spinnvlieses auf Basis eines Polymers, insbesondere Polyolefin, insbesondere Polypropylen, wobei zur Faserherstellung der Polymer als Rohmaterial in einem Extruder geschmolzen und unter hohem Druck durch eine Spinndüse gepresst wird, ist es besonders vorteilhaft, dass dem Rohmaterial vor dem Extrudieren im Extruder ein Anteil von 1 % bis 50 % eines Kunststoffadditivs enthaltend Zinksalz der Ricinolsäure beigemischt wird, insbesondere ein Kunststoffadditiv mit einem Gewichtsanteil von 10 % bis 20 %, insbesondere 15 % Zinksalz der Ricinolsäure.

Die erfindungsgemäßen Verfahren sind dabei unabhängig von dem zugrunde liegenden Herstellungs- und Spinnverfahren zur Herstellung der Fasern anwendbar, d.h. bei allen grundsätzlich bekannten Faserherstellungsverfahren zur Produktion von nichtgewebten Stoffen, Vliesen und Vliesstoffen, sogenannter Nonwoven. Insbesondere sind die Verfahren sowohl anwendbar auf das Feinfaserspinnvliesverfahren, bei dem die Faserverlängerung nach dem Pressen der Schmelze durch die Spinndüse im freien Fall erfolgt, als auch beim sogenannten Meltblown-Verfahren, bei dem die Faserverlängerung nach dem Pressen der Schmelze durch die Spinndüse in einem Heißluftstrom erfolgt.

Die erfindungsgemäßen Verfahren sind anwendbar bei allen Verfahren zur Faserherstellung, bei denen die Faserherstellung durch Schmelzspinnen erfolgt, insbesondere unabhängig davon, wie nach dem Pressen der Schmelze durch die Spinndüse die Streckung der Fasern erfolgt. Eine Streckung der Fasern nach dem Pressen der Schmelze durch die Spinndüse kann im freien Fall und/oder in einem Heißluftstrom nach dem sogenannten Meltblown-Verfahren erfolgen.

Durch die Erfindung ist es auf besonders einfache Weise möglich, dem Fasermaterial des Feinfaserspinnvlieses Zinksalz der Ricinolsäure als Geruchsabsorber in der gewünschten Menge zuzuführen, so dass das Fasermaterial nach Abschluss des Herstellungsverfahrens den gewünschten Anteil an Zinksalz der Ricinolsäure als Geruchsabsorber aufweist. Es wird somit zur Herstellung des Feinfaserspinnvlieses ein Kunststoffadditiv enthaltend einen Geruchsabsorber in Form von Zinksalz der Ricinolsäure bereitgestellt und in der entsprechenden Menge beigemischt, so dass das Endmaterial des Feinfaserspinnvlieses den gewünschten Gewichtsanteil an Zinksalz der Ricinolsäure aufweist, insbesondere einen Gewichtsanteil von 0,15 % bis zu 10 % Zinksalz der Ricinolsäure aufweist.

Zur Herstellung wird demnach einerseits das Rohmaterial zur Herstellung der Fasern bereitgestellt, beispielsweise Polypropylen. Ferner wird ein Kunststoffadditiv bereitgestellt, welches Zinksalz der Ricinolsäure in einer höheren Konzentration aufweist, als es das Endprodukt aufweisen soll. Das Rohmaterial und das Additiv werden dann in einem festgelegten Mischungsverhältnis dem Extruder zugeführt, sodass das Endprodukt, also die Fasern des Vlieses, den gewünschten Gewichtsanteil an Zinksalz der Ricinolsäure aufweist.

Es ist dabei beliebig, ob das Additiv, welches das Zinksalz der Ricinolsäure enthält, vor dem Einfüllen in den Extruder dem Rohmaterial oder erst im Extruder dem Rohmaterial beigegeben wird, da der Vorgang des Extrudierens eine optimale und homogene Materialmischung herbeiführt und das erzeugten Fasern eine homogene Mischung des Polymers, insbesondere Polypropylen, und des Zinksalzes der Ricinoisäure aufweisen.

Dabei hat sich gezeigt, dass die geruchsabsorbierende Wirkung mit steigendem Anteil an Zinksalz der Ricinolsäure an dem Material der Fasern des Vliesstoffes ansteigt. Mit steigendem Anteil an Zinksalz der Ricinolsäure an dem Material der Fasern des Vliesstoffes steigt sowohl die Wirksamkeit der Bindung unangenehmer Gerüche, als auch die mögliche Einsatzdauer des Vliesstoffes, bevor die Geruchsbindungswirkung merklich absinkt.

Besonders vorteilhaft ist dabei, dass das Material der Fasern des Vlieses eine homogene Verteilung des geruchsbindenden Wirkstoffes aufweisen, und hierdurch ein versehentliches Abwaschen oder Abtragen des Wirkstoffes von den Fasern vermieden wird, was bei lediglich beschichteten Fasern passieren kann, verbunden mit der Folge, dass die geruchsbindende Wirkung entfällt.

Bevorzugt werden dem Rohmaterial vor oder während des Extrudierens im Extruder Aromastoffe beigemischt, insbesondere ein Anteil von bis zu 1,5 % Aromastoffe.

Durch die Einbringung von Aromastoffen zusätzlich zu dem geruchsbindendem Zinksalz der Ricinolsäure kann ein Vliesstoff erzeugt werden, der einerseits unangenehme Gerüche durch das Binden der die Gerüche erzeugenden Stickstoff- und Schwefelverbindungen bindet, und gleichzeitig durch die im Material der Fasern des Vliesstoffes enthaltenen Aromastoffe ein angenehmes Aroma verströmt. Dabei können beliebige Aromen verwendet werden.

Sofern das Material des Vlieses gleichzeitig Aromastoffe aufweist, können hieraus gebildete Artikel wie beispielsweise Hygieneartikel gleichzeitig neben der Wirkung der Bindung unangenehmer Gerüche angenehme Aromen freisetzen und hierdurch eine deutlich angenehmere Anwendung für den Benutzer ermöglichen.

Vorzugsweise erfolgt nach dem Extrudieren und Spinnen der Fasern eine Verfestigung der Fasern durch Kalandrieren zwischen zwei Kalanderwalzen.

Dabei sind die Kalanderwalzen beheizt und es erfolgt ein punktuelles Aufschmelzen des Fasermaterials, wobei an den Kontaktpunkten die Fasern verschmelzen und der Vliesstoff verfestigt wird.

Hierdurch werden dem Spinnvlies die gewünschte Festigkeit und die gewünschten mechanischen Eigenschaften verliehen, sodass das Spinnvlies für eine Vielzahl von Anwendungen, beispielsweise als Filtermaterial, geeignet ist.

Das erfindungsgemäße Feinfaserspinnvlies ist somit aus Fasern gebildet, deren Material aus bis zu 10 % Gewichtsanteil Zinksalz der Ricinolsäure und bis zu 1,5 % Gewichtsanteil Aromastoffen bestehen, wobei der restliche Gewichtsanteil durch ein Polymer, insbesondere Polyolefin, bevorzugt Polypropylen, gebildet ist.

### Ausführungsbeispiele

### Beispiel 1:

Bei einem ersten Ausführungsbeispiel wurden dem Extruder Polypropylen als Rohmaterial und ein Zinksalz der Ricinolsäure enthaltendes Kunststoffadditiv in Granulatform im Verhältnis 9 zu 1 zugeführt, wobei das Kunststoffadditiv einen Anteil von 15 % an Zinksalz der Ricinolsäure aufwies. Das hierdurch hergestellte Feinfaserspinnvlies wies Fasern mit einer homogenen Zusammensetzung von 1,5 % Zinksalz der Ricinolsäure und 98,5 % Polypropylen auf und eignete sich besonders gut für eine Verwendung als Filter in Staubsaugerbeuteln, der gleichzeitig eine geruchsbindende Wirkung aufwies.

### Beispiel 2:

Bei einem zweiten Ausführungsbeispiel wurden dem Extruder Polypropylen als Rohmaterial und ein Zinksalz der Ricinolsäure enthaltendes Kunststoffadditiv in Granulatform sowie Aromastoffe zugeführt, wobei das Kunststoffadditiv einen Anteil von 15 % an Zinksalz der Ricinolsäure aufwies. Das hierdurch hergestellte Feinfaserspinnvlies wies Fasern mit einer homogenen Zusammensetzung von 3,2 % Zinksalz der Ricinolsäure und 96,8 % Polypropylen auf. Es zeigte sich eine gegenüber dem ersten Ausführungsbeispiel verbesserte geruchsbindende Wirkung des Vlieses.

### Beispiel 3:

Bei einem dritten Ausführungsbeispiel wurden dem Extruder Polypropylen als Rohmaterial und ein Zinksalz der Ricinolsäure enthaltendes Kunststoffadditiv in Granulatform im Verhältnis von 1 zu 1 zugeführt, wobei das Kunststoffadditiv einen Anteil von 15 % an Zinksalz der Ricinolsäure aufwies. Das hierdurch hergestellte Feinfaserspinnvlies wies Fasern mit einer homogenen Zusammensetzung von 8 % Zinksalz der Ricinolsäure 92 % Polypropylen auf und zeigte eine gegenüber dem zweiten Ausführungsbeispiel weiter gesteigerte geruchsbindende Wirkung.

### Beispiel 4:

Bei einem vierten Ausführungsbeispiel wurden dem Extruder Polypropylen als Rohmaterial und ein Zinksalz der Ricinolsäure enthaltendes Kunststoffadditiv in Granulatform im Verhältnis von 1 zu 1 zugeführt, wobei das Kunststoffadditiv einen Anteil von 15 % an Zinksalz der Ricinolsäure aufwies. Ferner wurden Aromastoffe in der Größenordnung von 1 % der Gesamtmenge von Rohmaterial und Additiv beigemischt. Das hierdurch hergestellte Feinfaserspinnvlies wies Fasern mit einer homogenen Zusammensetzung von 7,6 % Zinksalz der Ricinolsäure, 0,9 % Aromastoffe und 91,5 % Polypropylen auf und zeigte in etwa die selbe geruchsbindende Wirkung wie das dritte Ausführungsbeispiel.

### Beispiel 5:

Bei einem fünften Ausführungsbeispiel wurden dem Extruder Polypropylen als Rohmaterial und ein Zinksalz der Ricinolsäure enthaltendes Kunststoffadditiv in Granulatform im Verhältnis 1 zu 1 zugeführt, wobei das Kunststoffadditiv einen Anteil von 20 % an Zinksalz der Ricinolsäure aufwies. Das hierdurch hergestellte Feinfaserspinnvlies wies Fasern mit einer homogenen Zusammensetzung von 10 % Zinksalz der Ricinolsäure und 90 % Polypropylen auf und eignete sich aufgrund des relativ hohen Anteils an Zinksalz der Ricinolsäure besonders gut für eine Verwendung als Bestandteil in Hygieneartikeln wie Windeln, Binden, Tampons und dergleichen und zeigte eine sehr starke geruchsbindende Wirkung.

### Beispiel 6:

Bei einem sechsten Ausführungsbeispiel wurden dem Extruder Polypropylen als Rohmaterial und ein Zinksalz der Ricinolsäure enthaltendes Kunststoffadditiv in Granulatform im Verhältnis 1 zu 1 sowie Aromastoffe zugeführt, wobei das Kunststoffadditiv einen Anteil von 20 % an Zinksalz der Ricinolsäure aufwies. Das hierdurch hergestellte Feinfaserspinnvlies wies Fasern mit einer homogenen Zusammensetzung von 9,9 % Zinksalz der Ricinolsäure, 1,0 % Aromastoffe und 89,1 % Polypropylen auf und eignete sich besonders gut für eine Verwendung als Bestandteil in Hygieneartikeln wie Windeln, Binden, Tampons und dergleichen, da zusätzlich zu der sehr starken geruchsbindenden Wirkung angenehme Aromastoffe freigesetzt wurden und der olfaktorische Eindruck hierdurch sehr vorteilhaft war.

Bei allen Ausführungsbeispielen ließen sich die hergestellten Fasern ohne Probleme mittels Kalanderwalzen weiterverarbeiten und verfestigen sowie zuschneiden und konfektionieren.

## Patentansprüche

1. Staubsaugerfilterbeutel, welcher aus einem mehrlagigen Verbundmaterial aufweisend mehrere Filterlagen gebildet ist und einen eingefassten Anschlussbereich aufweist, wobei zumindest eine Filterlage durch ein Feinfaserspinnvlies auf Basis eines Polymers, insbesondere Polyolefin, insbesondere Polypropylen, gebildet ist, wobei zur Faserherstellung der Polymer als Rohmaterial in einem Extruder erhitzt und unter hohem Druck durch eine Spinndüse gepresst wird, **dadurch gekennzeichnet, dass** dem Rohmaterial des Vlieses vor dem Extrudieren oder während des Extrudierens im Extruder ein Anteil von 1 % bis 50 % eines Kunststoffadditivs enthaltend Zinksalz der Ricinolsäure beigemischt wird, so dass das Material der Fasern des Vlieses Zinksalz der Ricinolsäure aufweist, wobei das Material der Fasern des Vlieses einen Gewichtsanteil von bis zu 10 % Zinksalz der Ricinolsäure aufweist.

2. Staubsaugerfilterbeutel nach Anspruch 1, **dadurch gekennzeichnet, dass** das Material der Fasern des Vlieses einen Gewichtsanteil von 0,1 % bis zu 10 %, insbesondere von 0,5 % bis zu 1,5 %, insbesondere von 1 % Zinksalz der Ricinolsäure aufweist.

3. Staubsaugerfilterbeutel nach einem der vorherigen Ansprüche, **dadurch gekennzeichnet, dass** das Material der Fasern des Vlieses Aromastoffe aufweist, insbesondere einen Gewichtsanteil von bis zu 1,5 % Aromastoffe aufweist.

4. Staubsaugerfilterbeutel nach einem der vorherigen Ansprüche, **dadurch gekennzeichnet, dass** nach dem Extrudieren und Spinnen eine Verfestigung der Fasern des Vlieses durch Kalandrieren zwischen zwei Kalanderwalze und/oder durch Kleben und/oder durch Ultraschallverschweißung erfolgt

5. Staubsaugerfilterbeutel nach einem der vorherigen Ansprüche, **dadurch gekennzeichnet, dass** das Verbundmaterial zumindest eines Grobfilterlage und eine Feinfilterlage aufweist.

6. Staubsaugerfilterbeutel nach einem der vorherigen Ansprüche, **dadurch gekennzeichnet, dass** der Anschlussbereich durch einen Kunststoffeinsatz oder einen Einsatz aus Fasermaterial, insbesondere Pappe, oder einen Verbund aus Kunststoff und Fasermaterial gebildet ist, an den das den Beutel bildende Verbundmaterial angeklebt und/oder kraftschlüssig geklemmt und/oder angeschweißt ist.

7. Verfahren zur Herstellung eines Spinnvlieses auf Basis eines Polymers, insbesondere Polyolefin, insbesondere Polypropylen, insbesondere zur Verwendung als Bestandteil in Staubsaugerfilterbeuteln, wobei zur Faserherstellung der Polymer als Rohmaterial in einem Extruder geschmolzen und unter hohem Druck durch eine Spinndüse gepresst wird, **dadurch gekennzeichnet, dass** dem Rohmaterial vor oder während des Extrudierens im Extruder ein Anteil von 1 bis 50 %, insbesondere ein Anteil von 10 % bis 20 % eines Kunststoffadditivs enthaltend Zinksalz der Ricinolsäure beigemischt wird, insbesondere ein Kunststoffadditiv mit einem Gewichtsanteil von 10 % bis 20 %, insbesondere 15 % Zinksalz der Ricinolsäure.

8. Verfahren nach Anspruch 7, **dadurch gekennzeichnet, dass** dem Rohmaterial vor oder während des Extrudierens im Extruder Aromastoffe beigemischt werden, insbesondere ein Anteil von bis zu 1,5 % Aromastoffe.

9. Verfahren nach einem der Ansprüche 7 oder 8, **dadurch gekennzeichnet, dass** eine Streckung der Fasern nach dem Pressen der Schmelze durch die Spinndüse im freien Fall entlang einer Fallstrecke und/oder in einem Heißluftstrom erfolgt.

10. Verfahren nach einem der Ansprüche 7 bis 9, **dadurch gekennzeichnet, dass**
nach dem Extrudieren und Spinnen eine Verfestigung der Fasern durch Kalandrieren zwischen zwei Kalanderwalzen erfolgt.

## Claims

1. Vacuum cleaner filter bag which is formed from a multi-layer composite material comprising a plurality of filter layers and which comprises an enclosed connection region, at least one filter layer being formed by a fine spunbonded nonwoven fabric based on a polymer, in particular polyolefin, in particular polypropylene, the polymer, as the raw material, being heated in an extruder and pressed, under pressure, through a spinning nozzle to produce fibres, **characterised in that** a proportion of from 1 % to 50 % of a plastics additive containing ricinoleic acid zinc salt is added to the raw material of the nonwoven fabric in the extruder prior to extrusion or during extrusion, so that the material of the fibres in the nonwoven fabric comprises ricinoleic acid zinc salt, the material of the fibres of the nonwoven fabric comprising up to 10 % by weight ricinoleic acid zinc salt.

2. Vacuum cleaner filter bag according to claim 1, **characterised in that** the material of the fibres in the nonwoven fabric comprises from 0.1 % to 10 %, in particular from 0.5 % to 1.5 %, in particular 1 % by weight ricinoleic acid zinc salt.

3. Vacuum cleaner filter bag according to either of the preceding claims, **characterised in that** the material of the fibres in the nonwoven fabric comprises flavouring agents, in particular up to 1.5 % by weight flavouring agents.

4. Vacuum cleaner filter bag according to any of the preceding claims, **characterised in that**, after extrusion and spinning, the fibres in the nonwoven fabric are strengthened by being calendered between two calender rolls and/or glued and/or ultrasound-welded.

5. Vacuum cleaner filter bag according to any of the preceding claims, **characterised in that** the composite material comprises at least one coarse filter layer and a fine filter layer.

6. Vacuum cleaner filter bag according to any of the preceding claims, **characterised in that** the connection region is formed by either a plastics insert or an insert made of fibrous material, in particular cardboard, or a composite of plastics material and fibrous material, to which insert the composite material forming the bag is adhered and/or force-locked and/or welded.

7. Method for producing a spunbonded nonwoven fabric based on a polymer, in particular polyolefin, in particular polypropylene, in particular for use as a component in vacuum cleaner filter bags, the polymer, as the raw material, being melted and pressed, under pressure, through a spinning nozzle to produce fibres, **characterised in that** a proportion of from 1 % to 50 %, in particular a proportion of from 10 % to 20 %, of a plastics additive containing ricinoleic acid zinc salt, in particular a plastics additive comprising from 10 % to 20 %, in particular 15 %, by weight ricinoleic acid zinc salt, is added to the raw material in the extruder prior to or during extrusion.

8. Method according to claim 7, **characterised in that** flavouring agents, in particular a proportion of up to 1.5 % flavouring agents, are added to the raw material in the extruder prior to or during extrusion.

9. Method according to either claim 7 or claim 8, **characterised in that** once the melt has been pressed through the spinning nozzle, the fibres are stretched in free fall along a drop path and/or in a hot air current.

10. Method according to any of claims 7 to 9, **characterised in that**, after extrusion and spinning, the fibres are strengthened by being calendered between two calender rolls.

## Revendications

1. Sac filtrant d'aspirateur, qui est formé d'un matériau composite multicouche présentant plusieurs couches filtrantes et d'une zone de raccordement sertie, dans lequel au moins une couche filtrante est formée d'un non tissé à fibres fines à base d'un polymère, en particulier une polyoléfine, notamment du polypropylène, dans lequel, pour produire les fibres, le polymère est chauffé à l'état de matériau brut dans une extrudeuse et refoulé sous pression élevée à travers une filière, **caractérisé en ce que** l'on mélange au matériau brut du non tissé, avant extrusion ou en cours d'extrusion dans l'extrudeuse, une fraction de 1 % à 50 % d'un additif de matière plastique contenant un sel de zinc de l'acide ricinoléique de sorte que le matériau des fibres du non tissé contienne du sel de zinc de l'acide ricinoléique, le matériau des fibres du non tissé comportant une fraction pondérale allant jusqu'à 10 % de sel de zinc de l'acide ricinoléique.

2. Sac filtrant d'aspirateur selon la revendication 1, **caractérisé en ce que** le matériau des fibres du non tissé comporte une fraction pondérale de 0,1 % à 10 %, en particulier de 0,5 % à 1,5 %, tout particulièrement de 1 % de sel de zinc de l'acide ricinoléique.

3. Sac filtrant d'aspirateur selon l'une quelconque des revendications précédentes, **caractérisé en ce que** le matériau des fibres du non tissé comporte des substances aromatiques, en particulier une fraction pondérale allant jusqu'à 1,5 % de substances aromatiques.

4. Sac filtrant d'aspirateur selon l'une quelconque des revendications précédentes, **caractérisé en ce que**, après extrusion et filage, on procède à une consolidation des fibres du non tissé par calandrage entre deux rouleaux de calandre et/ou par collage et/ou par soudage aux ultrasons.

5. Sac filtrant d'aspirateur selon l'une quelconque des revendications précédentes, **caractérisé en ce que** le matériau composite présente au moins une couche filtrante grossière et une couche filtrante fine.

6. Sac filtrant d'aspirateur selon l'une quelconque des revendications précédentes, **caractérisé en ce que** la zone de raccordement est formée d'une pièce rapportée en matière plastique ou d'une pièce rapportée en matériau fibreux, en particulier en carton, ou en composite de matière plastique et de matériau fibreux, à laquelle est collé et/ou comprimé à force et/ou soudé le matériau composite formant le sac.

7. Procédé de production d'un non tissé à base d'un polymère, en particulier une polyoléfine, notamment du polypropylène, en particulier pour utilisation comme constituant dans des sacs filtrants d'aspirateur, dans lequel, pour produire les fibres, le polymère est fondu à l'état de matériau brut dans une extrudeuse et refoulé sous pression élevée à travers une filière, **caractérisé en ce que** l'on mélange au matériau brut, avant extrusion ou en cours d'extrusion dans l'extrudeuse, une fraction de 1 % à 50 %, en particulier une fraction de 10 % à 20 % d'un additif de matière plastique contenant un sel de zinc de l'acide ricinoléique, tout particulièrement un additif de matière plastique avec une fraction pondérale de 10 % à 20 %, notamment de 15 % de sel de zinc de l'acide ricinoléique.

8. Procédé selon la revendication 7, **caractérisé en ce que** l'on ajoute au matériau brut, avant ou pendant l'extrusion dans l'extrudeuse, des substances aromatiques, en particulier une fraction allant jusqu'à 1,5 % de substances aromatiques.

9. Procédé selon la revendication 7 ou la revendication 8, **caractérisé en ce que** l'on procède à un étirage des fibres, après refoulement du bain fondu à travers la filière, en chute libre le long d'un parcours de chute et/ou dans un courant d'air chaud.

10. Procédé selon l'une quelconque des revendications 7 à 9, **caractérisé en ce que**, après extrusion et filage, on procède à une consolidation des fibres par calandrage entre deux rouleaux de calandre.
